# EUROPEAN PATENT SPECIFICATION

(11) **EP 4 090 327 B1**
(45) Date of publication and mention of the grant of the patent: **05.03.2025**
(21) Application number: 21700866.3
(22) Date of filing: 14.01.2021
(51) Int. Cl.: A61K 31/167, A61K 31/397, A61K 31/4045, A61K 31/4162, A61K 31/42, A61K 31/423, A61K 31/426, A61K 31/439, A61K 31/4439, A61K 31/4545, A61K 31/496, A61K 31/513, A61K 31/55, A61K 31/575, A61K 31/675, A61K 31/7056, A61K 31/7072, A61K 31/708, A61K 31/7088, A61K 38/21, A61P 31/14

(54) **USE OF FXR AGONISTS FOR TREATING AN INFECTION BY HEPATITIS D VIRUS**
VERWENDUNG VON FXR-AGONISTEN ZUR BEHANDLUNG EINER INFEKTION DURCH DAS HEPATITIS-D-VIRUS
UTILISATION D'AGONISTES DU FXR POUR LE TRAITEMENT D'UNE INFECTION PAR LE VIRUS DE L'HÉPATITE D

(30) Priority: 15.01.2020 EP 20305024
(43) Date of publication of application: 23.11.2022
(73) Proprietor: INSERM (Institut National de la Santé et de la Recherche Médicale), 75013 Paris (FR); Ecole Normale Supérieure de Lyon, 69007 Lyon (FR); Université Claude Bernard Lyon I, 69622 Villeurbanne Cedex (FR); Centre National de la Recherche Scientifique, 75016 Paris (FR); ENYO Pharma, 69008 Lyon (FR)
(72) Inventor: DARTEIL, Raphaël, 69006 LYON (FR); LUCIFORA, Julie, 69003 LYON (FR); DURANTEL, David, 69740 GENAS (FR); RAMIERE, Christophe, 69007 LYON (FR); LACOMBE, Benoît, 1020 Renens VD (CH); LOTTEAU, Vincent, 69390 VOURLES (FR); ANDRE, Patrice, 69003 LYON (FR)
(74) Representative: Cabinet Becker et Associés
(86) International application number: PCT/EP2021/050625
(87) International publication number: WO 2021/144330

(56) References cited:
- ZHIMIN GUO ET AL: "Therapeutic Strategies and New Intervention Points in Chronic Hepatitis Delta Virus Infection", INTERNATIONAL JOURNAL OF MOLECULAR SCIENCES, vol. 16, no. 8, 18 August 2015 (2015-08-18), pages 19537 - 19552, XP055245823, DOI: 10.3390/ijms160819537
- CIHAN YURDAYDIN ET AL: "Therapy of Delta Hepatitis", COLD SPRING HARBOR PERSPECTIVES IN MEDICINE, vol. 5, no. 10, 7 August 2015 (2015-08-07), pages a021543, XP055711218, DOI: 10.1101/cshperspect.a021543
- NIDHI SINGH ET AL: "Synthetic FXR Agonist GW4064 Is a Modulator of Multiple G Protein-Coupled Receptors", MOLECULAR ENDOCRINOLOGY, vol. 28, no. 5, 1 May 2014 (2014-05-01), pages 659 - 673, XP055207727, ISSN: 0888-8809, DOI: 10.1210/me.2013-1353
- DAVID C. TULLY ET AL: "Discovery of Tropifexor (LJN452), a Highly Potent Non-bile Acid FXR Agonist for the Treatment of Cholestatic Liver Diseases and Nonalcoholic Steatohepatitis (NASH)", JOURNAL OF MEDICINAL CHEMISTRY, vol. 60, no. 24, 16 November 2017 (2017-11-16), US, pages 9960 - 9973, XP055551278, ISSN: 0022-2623, DOI: 10.1021/acs.jmedchem.7b00907
- E. THOMAS ET AL: "Viral Hepatitis: Past and Future of HBV and HDV", COLD SPRING HARBOR PERSPECTIVES IN MEDICINE, vol. 5, no. 2, 1 February 2015 (2015-02-01), pages a021345 - a021345, XP055711802, DOI: 10.1101/cshperspect.a021345

## Description

### FIELD OF THE INVENTION

The present invention relates to the field of medicine, especially hepatology and virology, more particularly the treatment of infection by Hepatitis D virus (HDV) in a subject suffering from a chronic HDV infection.

### BACKGROUND OF THE INVENTION

Hepatitis D virus (HDV) infection is the most severe form of chronic viral hepatitis due to rapid progression towards liver-related death and hepatocellular carcinoma. The World Health Organization (WHO) estimates that 15-20 million persons are infected by HDV (*www.who.int*/*en*/*news-room*/*fact-sheets*/*detail*/*hepatitis-d*). The most recent meta-analysis of HDV burden suggests an underestimation of hepatitis D prevalence; indeed, seroprevalence might be as high as 0.98% of the worldwide population and 10.58% of global chronic hepatitis B patients (Chen H-Y et al. Gut 2019;68:512-521). In the absence of specific anti-HDV therapy, current guidelines generally recommend subcutaneous injection of Pegylated-interferon-alpha-2a (PEG-IFN-α2a), which is a non-specific immune-stimulator, rather toxic and poorly supported by patients. It is often used in addition to a nucleoside analogue, which controls HBV viremia. The overall rate of sustained virological response is low. Indeed, a response rate on treatment of about 25% has been reported and a high level of relapse after cessation of treatment. Accordingly, there is an unmet need of new therapeutic agents and strategies for the treatment of infection by HDV. However, there are several difficulties in the development of therapeutic agents for these patients. HDV genome is a single-stranded RNA (≈ 1700 nucleotides) of negative polarity containing a single open reading frame encoding two viral proteins: the small and the large delta antigens (HDAg-S and HDAg-L). Replication of the HDV RNA genome takes place in the nucleus of infected cells, and occurs by a rolling circle mechanism, followed by cleavage by endogenous ribozymes and ligations resulting in the formation of antigenomic circular monomers. From these circular antigenomic monomers, the same mechanisms generate new genomic circular RNA monomers. It is assumed that HDV hijacks DNA-dependent host RNA-polymerase(s) during the genome replication steps. HDV uses at least the RNA polymerase II for both replication and transcription of viral mRNA but the role of RNA polymerase I and III is also suggested *(*Mentha N et al. J Adv Res. 2019 May; 17:3-15*).* During this process, viral linear mRNAs are also synthesized resulting in synthesis of HDAg-S and HDAg-L. Compared with HDAg-S (195 amino acids), HDAg-L (214 amino acids) contains an additional domain of 19-20 amino acids at its C-terminus resulting from ADAR-1-mediated RNA editing of the antigenomic HDV RNA at a location corresponding to the stop codon of HDAg-S gene (Wong SK, Lazinski DW. Proc Natl Acad Sci U S A. 2002 Nov 12;99(23):15118-23). HDAg-S is involved in HDV accumulation during the replication step. In particular, HDAg-S is thought to interact with numerous cellular proteins (more than 100 identified interactants) and is present in a nuclear complex in association with cellular proteins involved in transcriptional regulation such as Yin Yang 1 (YY1), Histone acetyltransferase (HAT) p300 Creb Binding Protein (p300/CBP) and selectivity factor 1 (SL1), which belongs to the pre-initiation complex of RNA polymerase I (Huang W-H etal. J Virol. 2008 Aug;82(15):7313-24*;* Li Y-Jet al. J Virol. 2006 Jul;80(13):6478-86*).* Interaction of HDAg-S with cellular histone H1.4 has also been described with consequences on viral replication (Lee C-Z, Sheu J-C. Virology. 2008 May 25;375(1):197-204). Whereas HDAg-S is mainly involved in the replication step, HDAg-L is essential for virion budding. The 19-20 amino acids additional domain in HDAg-L contains a CXXX-box motif, a substrate for cellular farnesyltransferase, which adds a farnesyl group to the cysteine of this CXXX-box. This farnesylation process was shown to be essential for virion assembly (Glenn J et al. Science. 1992 May 29;256(5061):1331-3*).* During this step, HDV hijacks HBV surface antigens for its own use and the secretion of infectious HDV virions that can spread or maintain the infection. HBV and HDV virions contain the same envelope proteins and are undistinguishable from a humoral-response perspective. Consequently, HBV and HDV share the same entry receptor, i.e. sodium taurocholate cotransporting polypeptide (NTCP), the main transporter of bile acid (BA) at the baso-lateral membrane of hepatocytes *(*Yan H et al. eLife [Internet]. 2012 Nov 13 [cited 2019 Sep 3];1. Available from: https://elifesciences.org/articles/00049*).* As a consequence of this viral symbiosis, HDV transmission generally occurs through HBV co-infection or super-infection. However, aside from the crucial role of HB antigens (HBAgs) for hepatocyte entry, the other steps of HDV life cycle described above, in particular the replication process, are not dependent on HBV and contribute per se to the severity of the disease and rapid evolution toward cirrhosis and HCC. In addition, in the typical course of HDV super-infection, markers of HBV infection are usually inhibited, with IgM anti-HBc and HBV DNA that could test negative *(*Romeo R, Perbellini R. World J Hepatol 2015;7:2389-95*;* Schaper M et al. J Hepatol. 2010;52:658-64*).* HBV replication is, however, usually suppressed to low levels during the acute phase of HDV infection. This suppression becomes persistent in case of a chronic hepatitis D establishment.

The co-infection with HDV and HBV is a complex situation that mixes features specific for each virus and some that are common to both. Importantly, recent studies report that addition of the standard of care for HBV treatment, which inhibits the HBV specific polymerase by nucleotide analogues, to HDV standard of care treatment with PEG-IFN-2a does not improve HDV response rate at the end of treatment *(*Wedemeyer H et al. Lancet Infect. Dis. 2019;19:275-286*;* Mentha N et al. J. Adv. Res. 2019;17:3-17*).* These findings clearly highlight that alternative treatment options that target HDV replication steps are needed for hepatitis D. Indeed, lonafarnib, an inhibitor of the enzyme farnesyl transferase, which is a mandatory step in virion assembly, repress HDV replication independently of HBV (application US20110129549A1; Mentha N, Cints.google.com/patAlfaiate D. J. Adv. Res. 2019;17:3-17)*.* However, its toxicity prevents its broad use in anti-HDV therapy. Several other antiviral molecules targeting the HDV interaction with the HBV HBsAg are currently under development: Myrcludex B, which blocks HDV entry into hepatocytes by inhibiting HBsAg binding to NTCP, siRNA silencing HBV mRNA, including HBsAg mRNA, and REP 2139, which is thought to inhibit HBsAg release from hepatocytes and interact with hepatitis delta antigen (Ye X et al. ACS Infect. Dis. 2019;5:738-5:7*;* Mentha N et al. J. Adv. Res. 2019;17:3-17)*.* Then, anti-HDV treatment should inhibit at least one HDV replication step. The replication step may be HDV specific, so as the prenylation of HDAg-L, or shared with HBV by inhibiting HBsAg synthesis, release, or function. Ideally, treatment of hepatitis D should not only repress HDV replication but also HBV replication by inhibiting specific replication steps of each virus. Besides compounds targeting the common HBsAg dependency of both viruses, no such molecule that could specifically repress both HDV replication *AND* HBV replication has been reported. Molecules inhibiting the two viruses are the subject of active research since it is difficult to predict how the second virus will react when a treatment is targeting only one virus. There is a risk of activation or reactivation of one virus when the other is inhibited. For instance, it has been observed in the development of antiviral agents against HCV that, in patients co-infected with HBV, reactivation of HBV has occurred when HCV was targeted (Ma et al, Gastroenterology, 2018, 154, 795-798). Therefore, patients co-infected by HBV and HDV are generally excluded from clinical trials. Indeed, a review in 2016 reports that less than 1000 patients coinfected with HDV were included in the clinical trials (Guglielmi S et al, 2016, Revue médicale Suisse, 12, 1415-1418).

ZHIMIN GUO ET AL ("Therapeutic Strategies and New Intervention Points in Chronic Hepatitis Delta Virus Infection", INTERNATIONAL JOURNAL OF MOLECULAR SCIENCES, vol. 16, no. 8, 18 August 2015, pages 19537-19552) concerns the treatment of co-infections of hepatitis D and B.

CIHAN YURDAYDIN ET AL. ("Therapy of Delta Hepatitis", COLD SPRING HARBOR PERSPECTIVES IN MEDICINE, vol. 5, no. 10, 7 August 2015 (2015-08-07), page a021543) discloses *inter alia* that acute delta hepatitis resembles a typical self-limited hepatitis that is clinically and histologically indistinguishable from hepatitis B.

### SUMMARY OF THE INVENTION

The inventors identified the capacity of several FXR agonists to prevent HDV RNA genome replication, in models of HDV mono-infection. This result is particularly surprising because, if FXR agonists are known inhibitors of HBV cccDNA formation and transcription (Mouzannar K et al. FASEB J. 2018;33:2472-33:2), the effect on HDV is independent of the presence of HBV. FXR agonists mechanism of action on HDV is therefore new and original, acting on specific HDV replication steps. In addition, the inventors demonstrated the capacity of several FXR agonists to inhibit the production of the two HDV proteins, namely the short hepatitis D antigen (HDAg-S) and the long hepatitis D antigen (HDAg-L). In models of HBV and HDV co-infection, the inventors showed that FXR agonists repressed both viruses' replication and production. Then, these findings open a new way for treating hepatitis D infection, in particular chronic hepatitis D infection, with a new antiviral class of molecules that inhibits specifically and independently HDV and HBV replication.

The present invention relates to a farnesoid X receptor (FXR) agonist for use for the treatment of hepatitis D virus (HDV) infection in a subject suffering from a chronic HDV infection.

Optionally, the FXR agonist is a selective FXR agonist.

In a particular aspect, the FXR agonist is selected from the group consisting of UN452 (Tropifexor), LMB763 (Nidufexor), GS-9674 (Cilofexor), PX-102 (PX-20606), PX-104 (Phenex 104), OCA (Ocaliva), EDP-305, TERN-101 (LY2562175), MET-409, GW4064, WAY362450 (Turofexorate isopropyl), Fexaramine, AGN242266 (AKN-083), BAR502, and EYP001.

In a very particular aspect, the FXR agonist is EYP001.

Optionally, the FXR agonist is for use in combination with an interferon alpha (IFN-α), an interferon lambda or a pegylated form thereof, preferably selected from the group consisting of IFN-α1a, IFN-α1b, IFN-α2a, IFN-α2b, and IFN-λ1a or a pegylated form thereof, more preferably PEG-IFN-α2a (e.g., Pegasys), PEG-IFN-α2b (e.g., ViraferonPeg or Introna) or PEG-IFN-λ1a.

Optionally, the FXR agonist is for use in combination with an anti-HDV agent, preferably a nucleoside analog or a farnesyl transferase inhibitor. In a particular aspect, said anti-HDV agent is selected from the group consisting of ribavirin, ritonavir, lonafarnib and EBP 921.

Optionally, the FXR agonist is for use in combination with an anti-HBV agent, preferably a nucleoside analog. In a particular aspect, said nucleoside analog is selected from the group consisting of lamivudine, adefovir, telbivudine, entecavir, tenofovir and emtricitabine.

Optionally, the FXR agonist is for use in combination with an anti-HBV/HDV agent, preferably a nucleoside analog, a nucleic acid polymer or a NTCP inhibitor. In a particular aspect, said anti-HBV/HDV agent is selected from the group consisting of ezetimibe, myrcludex B, nucleic acid polymer REP 2139 and nucleic acid polymer REP 2165.

Optionally, the subject has failed to respond to a previous treatment for HDV infection. In a first aspect, the previous treatment is a treatment with PEG-IFNα. In another particular aspect, the previous treatment is a treatment with an anti-HDV agent as defined in the claims.

### DETAILED DESCRIPTION OF THE INVENTION

The present invention relates to farnesoid X receptor (FXR) agonists for use in the treatment of hepatitis D virus (HDV) infection in a subject suffering from a chronic HDV infection.

The present invention relates to a pharmaceutical composition comprising a FXR agonist for use in the treatment of hepatitis D virus (HDV) infection in a subject suffering from a chronic HDV infection.

The invention is defined in the claims. Any subject-matter which is outside the scope of the claims is provided for reference or comparison only.

Any reference to a method of treatment of the human or animal body by therapy using a certain compound or composition is to be understood as relating to said compound or composition for use in said treatment.

### Patients

As used herein a "Hepatitis D virus infected patient" means a patient being infected with any Hepatitis B virus genotype, e.g., genotype 1, 2, 3, 4, 5, 6, 7, 8.

According to the invention, the term "subject" or "patient" and "subject in need thereof" or "patient in need thereof", is intended for a human or non-human mammal infected or likely to be infected with a hepatitis D virus. According to the claimed invention, the subject suffers from a chronic HDV infection.

The terms "coinfected patients" refers to individuals that have been simultaneously infected with HBV and HDV. The terms "super-infected patients" refers to individuals that have been firstly infected with HBV, and then infected with HDV.

According to an aspect of the invention, the terms "treatment failure patients" refers to individuals who have failed previous treatment for HDV infection. Consequently, "Treatment failure patients" as used herein generally refers to HDV-infected patients who failed to respond to the treatment (referred to as "non-responders") or who initially responded to the treatment, but in whom the therapeutic response was not maintained (referred to as "relapsers").

More specifically, although there is no effective treatment against HDV, or any FDA-approved drug for the treatment of chronic HDV, the pegylated immune system modulator interferon alpha (PEG-IFN-α) is currently used in clinical practice and thus considered as a standard-of-care for HDV infection. Consequently, "Treatment failure patients" as used herein generally refers to HDV-infected patients who failed to respond to the PEG-IFN-α treatment (referred to as "non-responders") or who initially responded to PEG-IFN-α treatment, but in whom the therapeutic response was not maintained (referred to as "relapsers").

### Treatment

As used herein, the term "treatment" or "treat" refer to both prophylactic or preventive treatment as well as curative or disease modifying treatment, including treatment of patient at risk of contracting the disease or suspected to have contracted the disease as well as patients who are ill or have been diagnosed as suffering from a disease or medical condition, and includes suppression of clinical relapse. The treatment may be administered to a subject having a medical disorder or who ultimately may acquire the disorder, in order to prevent, cure, delay the onset of, reduce the severity of, or ameliorate one or more symptoms of a disorder or recurring disorder, or in order to prolong the survival of a subject beyond that expected in the absence of such treatment.

The efficacy of the treatment may be monitored using standard protocols. Indeed, treatment may be followed by determinations of HDV levels in serum (viral load) and measurement of serum alanine aminotransferase (ALT) levels. For example, the patients may be assessed for the presence of HDV RNA in their serum. HDV RNA (IU/mL) can be measured at regular intervals during the treatment, e.g., at Day 1 (pre-dose and 4, 8, and 12 hours post-dose) and pre-dose at Day 2, Day 3, Day 8, Day 15, Day 29, and at Week 12, Week 24, Week 36, Week 48, Week 72 (when applicable), and at follow up. Accordingly, the efficacy of treatment can be monitored using internationally accepted parameters: a) Serum HDV RNA levels are monitored using sensitive quantitative RT-PCR-based assays to assess the effect on viral replication. b) Serum levels of ALT and/or aspartate aminotransferase (AST) are monitored to assess impact on liver inflammation and liver cell death.

The treatment may correspond to a single-agent treatment where only one FXR agonist is administered, or to a combination therapy with another therapeutic agent such as another FXR agonist or antiviral agents.

The treatment can be administered to individuals who have been diagnosed with an HDV infection. Any of the above treatment regimens can be administered to individuals who have failed previous treatment for HDV infection (treatment failure patients).

### FXR agonist

The term "FXR" refers to the farnesoid X receptor, which is a nuclear receptor that is activated by supraphysiological levels of farnesol (Forman et al., Cell, 1995,81,687-693). FXR, is also known as NR1H4, retinoid X receptor-interacting protein 14 (RIP14) and bile acid receptor (BAR). Containing a conserved DNA-binding domain (DBD) and a C-terminal ligand-binding domain (LBD), FXR binds to and becomes activated by a variety of naturally occurring bile acids (BAs), including the primary bile acid chenodeoxycholic acid (CDCA) and its taurine and glycine conjugates. Upon activation, the FXR-RXR heterodimer binds the promoter region of target genes and regulates the expression of several genes involved in bile acid homeostasis. Hepatic FXR target genes fall into two main groups. The first group functions to decrease hepatic bile acids concentrations by increasing export and decreasing their synthesis. The second group of FXR target genes such as the phospholipid transport protein PLTP and apolipoproteins modulates lipoprotein levels in the serum and decreases plasma triglyceride concentration. For a more detailed list of FXR-regulated genes, see, e.g., WO 03/016288, pages 22-23. US patent 6,005, 086 discloses the nucleic acid sequence coding for a mammalian FXR protein. The human polypeptide sequences for FXR are deposited in nucleotide and protein databases under accession numbers NM_005123, Q96RI1, NP_005114 AAM53551, AAM53550, AAK60271.

In this specification, the term "FXR agonist" has its general meaning in the art and refers in particular to compounds that function by targeting and binding the farnesoid X receptor (FXR) and which activate FXR by at least 40% above background in the assay described in Maloney et al. (J. Med. Chem. 2000, 43:2971-2974).

In some embodiments, the FXR agonist of the invention is a selective FXR agonist. As used herein, the term "selective FXR agonist" refers to an FXR agonist that exhibits no significant cross-reactivity to one or more, ideally substantially all, of a panel of nuclear receptors consisting of LXRα, LXRβ, PPARα, PPARγ, PPARδ, RXRα, RARy, VDR, PXR, ERα, ERβ, GR, AR, MR and PR. Methods of determining significant cross-reactivity are described in J. Med. Chem. 2009, 52, 904-907.

FXR agonists are well known to the skilled person.

For example, the skilled person may easily identify FXR agonist from the following publications:
Abenavoli L, et al. Pharmaceuticals (Basel). 2018 Oct 11;11(4). pii: E104. doi: 10.3390/ph11040104. Review.
Adorini L, et al. Drug Discov Today. 2012 Sep;17(17-18):988-97. doi: 10.1016/j.drudis.2012.05.012. Epub 2012 May 29. Review.
Akwabi-Ameyaw A, et al. Bioorg Med Chem Lett. 2009 Aug 15;19(16):4733-9. doi: 10.1016/j.bmcl.2009.06.062. Epub 2009 Jun 21.
Akwabi-Ameyaw A, et al. Bioorg Med Chem Lett. 2008 Aug 1;18(15):4339-43. doi: 10.1016/j.bmcl.2008.06.073. Epub 2008 Jun 28.
Akwabi-Ameyaw A, et al. Bioorg Med Chem Lett. 2011 Oct 15;21(20):6154-60. doi: 10.1016/j.bmcl.2011.08.034. Epub 2011 Aug 11.
Baghdasaryan A, et al. Hepatology. 2011 Oct;54(4):1303-12. doi: 10.1002/hep.24537.
Bass JY, et al. Bioorg Med Chem Lett. 2009 Jun 1;19(11):2969-73. doi: 10.1016/j.bmcl.2009.04.047. Epub 2009 Apr 18.
Bass JY, et al. Bioorg Med Chem Lett. 2011 Feb 15;21(4):1206-13. doi: 10.1016/j.bmcl.2010.12.089. Epub 2010 Dec 23.
Buijsman et al., Curr. Med. Chem. 2005, 12, 1017
Carino et al, Sci Rep. 2017 Feb 16;7:42801. doi: 10.1038/srep42801.
Chiang PC, et al. J Pharm Sci. 2011 Nov;100(11):4722-33. doi: 10.1002/jps.22664. Epub 2011 Jun 9. Crawley, Expert Opin. Ther. Pat. 2010, 20, 1047
Feng S, et al. Bioorg Med Chem Lett. 2009 May 1;19(9):2595-8. doi: 10.1016/j.bmcl.2009.03.008. Epub 2009 Mar 9.
Festa et al, Front Pharmacol. 2017 Mar 30;8:162. doi: 10.3389/fphar.2017.00162. eCollection 2017. Finamore et al, Sci Rep. 2016 Jul 6;6:29320. doi: 10.1038/srep29320.
Flatt B, et al. J Med Chem. 2009 Feb 26;52(4):904-7. doi: 10.1021/jm8014124.
Gege et al, Curr Top Med Chem. 2014;14(19):2143-58.
Gege et al, Handbook of Experimental Pharmacology, doi: 10.1007/164_2019_232..
Genin et al, J Med Chem. 2015 Dec 24;58(24):9768-72. doi: 10.1021/acs.jmedchem.5b01161. Epub 2015 Dec 2.
Ghebremariam YT, et al. PLoS One. 2013 Apr 4;8(4):e60653. doi: 10.1371/journal.pone.0060653. Print 2013.
Gioiello A, et al. Bioorg Med Chem. 2011 Apr 15;19(8):2650-8. doi: 10.1016/j.bmc.2011.03.004. Epub 2011 Mar 10.
Hoekstra M, et al. Mol Cell Endocrinol. 2012 Oct 15;362(1-2):69-75. doi: 10.1016/j.mce.2012.05.010. Epub 2012 May 27.
Iguchi Y, et al. Steroids. 2010 Jan;75(1):95-100. doi: 10.1016/j.steroids.2009.11.002. Epub 2009 Nov 12. Kinzel et al, Bioorg Med Chem Lett. 2016 Aug 1;26(15):3746-53. doi: 10.1016/j.bmcl.2016.05.070. Epub 2016 May 24.
Lin HR. Bioorg Med Chem Lett. 2012 Jul 15;22(14):4787-92. doi: 10.1016/j.bmcl.2012.05.057. Epub 2012 May 23.
Lundquist JT, et al. J Med Chem. 2010 Feb 25;53(4):1774-87. doi: 10.1021/jm901650u.
Ma Y, et al. Pharm Res. 2013 May;30(5):1447-57. doi: 10.1007/s11095-013-0986-7. Epub 2013 Feb 1.
Marinozzi M, et al. Bioorg Med Chem. 2013 Jul 1;21(13):3780-9. doi: 10.1016/j.bmc.2013.04.038. Epub 2013 Apr 23.
Massafra et al. Pharmacol Ther. 2018 Nov;191:162-177. doi: 10.1016/j.pharmthera.2018.06.009. Epub 2018 Jun 20.
Misawa T, et al. Bioorg Med Chem Lett. 2012 Jun 15;22(12):3962-6. doi: 10.1016/j.bmcl.2012.04.099. Epub 2012 Apr 30.
Pellicciari et al, J Med Chem. 2016 Oct 4.
Richter HG, et al. Bioorg Med Chem Lett. 2011 Feb 15;21(4):1134-40. doi: 10.1016/j.bmcl.2010.12.123. Epub 2010 Dec 31.
Rizzo G, et al. Mol Pharmacol. 2010 Oct;78(4):617-30. doi: 10.1124/mol.110.064501. Epub 2010 Jul 14.
Roda et al, J Pharmacol Exp Ther. 2014 Jul;350(1):56-68. doi: 10.1124/jpet.114.214650. Epub 2014 May 1.
Schuster D, et al. Bioorg Med Chem. 2011 Dec 1;19(23):7168-80. doi: 10.1016/j.bmc.2011.09.056. Epub 2011 Oct 4.
Schwabl et al, J Hepatol. 2017 Apr;66(4):724-733. doi: 10.1016/j.jhep.2016.12.005. Epub 2016 Dec 18.
Samlley et al, Bioorg Med Chem Lett. 2015 Jan 15;25(2):280-4. doi: 10.1016/j.bmcl.2014.11.050. Epub 2014 Nov 26.
Sepe et al. Expert Opin Ther Pat. 2018 May;28(5):351-364. doi: 10.1080/13543776.2018.1459569. Epub 2018 Apr 13. Review.
Sepe et al. Expert Opin Ther Pat. 2015;25(8):885-96. doi: 10.1517/13543776.2015.1045413. Review.
Soisson SM, et al. Proc Natl Acad Sci U S A. 2008 Apr 8;105(14):5337-42. doi: 10.1073/pnas.0710981105. Epub 2008 Apr 7.
Townsend SA, Newsome PN. Aliment Pharmacol Ther. 2017 Sep;46(5):494-507. doi: 10.1111/apt.14210. Epub 2017 Jul 4.
Tully et al, J Med Chem. 2017 Dec 28;60(24):9960-9973. doi: 10.1021/acs.jmedchem.7b00907. Epub 2017 Dec 8.
Wang et al, J Am Soc Nephrol. 2018 Jan;29(1):118-137. doi: 10.1681/ASN.2017020222. Epub 2017 Oct 31. Wang et al, Bioorg Med Chem Lett. 2017 Aug 1;27(15):3386-3390. doi: 10.1016/j.bmcl.2017.06.003. Epub 2017 Jun 3.
Wang H, et al. Expert Opin Ther Pat. 2018 Nov;28(11):765-782. doi: 10.1080/13543776.2018.1527906. Epub 2018 Oct 8. Review
Watanabe M, et al. J Biol Chem. 2011 Jul 29;286(30):26913-20. doi: 10.1074/jbc.M111.248203. Epub 2011 Jun 1.
Yu D, et al. Steroids. 2012 Nov;77(13):1335-8. doi: 10.1016/j.steroids.2012.09.002. Epub 2012 Sep 21. Zhang S, et al. J Hepatol. 2009 Aug;51(2):380-8. doi: 10.1016/j.jhep.2009.03.025. Epub 2009 May 18.
Typically, FXR agonists include the class of steroid FXR agonists and non-steroid FXR agonists.

In certain embodiments of the invention, the FXR agonist is selected from small molecule compounds which act as FXR modulators that have been disclosed in the following publications: EP1392714; EP1568706; JP2005281155; US20030203939; US2005080064; US2006128764; US20070015796; US20080038435; US20100184809; US20110105475; US6,984,560; WO2000037077; WO200040965; WO200076523; WO2003015771; WO2003015777; WO2003016280; WO2003016288; WO2003030612; WO2003016288; WO2003080803; WO2003090745; WO2004007521; WO2004048349; WO2004046162; WO2004048349; WO2005082925; WO2005092328; WO2005097097; WO2007076260; WO2007092751; WO2007140174; WO2007140183; WO2008002573; WO2008025539; WO2008025540; WO200802573; WO2008051942; WO2008073825; WO2008157270; WO2009005998; WO2009012125; WO2009027264; WO2009080555; WO2009127321; WO2009149795; WO2010028981; WO2010034649; WO2010034657; WO2017218330; WO2017218379; WO2017201155; WO2017201152; WO2017201150; WO2017189652; WO2017189651; WO2017189663; WO2017147137; WO2017147159; WO2017147174; WO2017145031; WO2017145040; WO2017145041; WO2017133521; WO2017129125; WO2017128896; WO2017118294; WO2017049172; WO2017049176; WO2017049173; WO2017049177; WO2016173397; WO2016173493; WO2016168553; WO2016161003; WO2016149111; WO2016131414; WO2016130809; WO2016097933; WO2016096115; WO2016096116; WO2016086115; WO2016073767; WO2015138986; WO2018152171; WO2018170165, WO2018170166, WO2018170173, WO2018170182, WO2018170167; WO2017078928; WO2014184271; WO2013007387; WO2012087519; WO2011020615; WO2010069604; WO2013037482; US2017275256; WO2005080064; WO2018190643; WO2018215070; WO2018215610; WO2018214959; WO2018081285; WO2018067704; WO2019007418; WO2018059314; WO2017218337; WO2020231917; WO2020211872; WO2020168143; WO2020168148; WO2020156241; WO2020150136; WO2020114307; WO2020061118; WO2020061114; WO2020061112; WO2020061113; WO2020061116, WO2020061117; WO2020011146; WO2020001304; WO2019160813; WO2019120088; WO2019118571; WO2019089667; WO2019089672; WO2019089665; WO2019089664; WO2019089670.

In an aspect, the FXR agonist can be any FXR agonists disclosed in the following patent applications: WO2017/049172, WO2017/049176, WO2017/049173, WO2017/049177, WO2018/170165, WO2018/170166, WO2018/170173, WO2018/170182, and WO2018/170167.

Specific examples of FXR agonists include but are not limited to EYP001, GW4064 (as disclosed in PCT Publication No. WO 00/37077 or in US2007/0015796), 6 -ethyl-chenodeoxycholic acids, especially 3α, 7α-dihydroxy 7α-dihydroxy-6α-ethyl-5β-cholan-24-oic acid, also referred to as INT-747; INT-777; 6 -ethyl-ursodeoxycholic acids, INT-1103, UPF-987, WAY-362450, MFA-1, GW9662, T0901317, fexaramine, 3β-azido-6α-ethyl-7α-hydroxy-5β-cholan-24-oic acid, Tropifexor (LJN452), fexaramine-3 (Fex-3), BAR502, BAR704, PX20606, PX20350, 3α,7α,11β-Trihydroxy-6α-ethyl-5β-cholan-24-oic Acid (TC-100), 6-(4-{[5-Cyclopropyl-3-(2,6-dichlorophenyl)isoxazol-4-yl]methoxy}piperidin-1-yl)-1-methyl-1H-indole-3-carboxylic Acid, 3,6-dimethyl-1-(2-methylphenyl)-4-(4-phenoxyphenyl)-4,8-dihydro-1H-pyrazolo[3,4-e][1,4]thiazepin-7-one; obeticholic acid, a cholic acid, a deoxycholic acid, a glycocholic acid, a glycodeoxycholic acid, a taurocholic acid, a taurodihydrofusidate, a taurodeoxycholic acid, a cholate, a glycocholate, a deoxycholate, a taurocholate, a taurodeoxycholate, a chenodeoxycholic acid, an ursodeoxycholic acid, a tauroursodeoxycholic acid, a glycoursodeoxycholic acid, a 7-B-methyl cholic acid, a methyl lithocholic acid, GSK-8062 (CAS No. 943549-47-1). In some embodiments, the FXR agonist is selected from natural bile acids, preferably chenodeoxycholic acid [CDCA] or taurine- or glycine-conjugated CDCA [tauro-CDCA or glyco-CDCA] and synthetic derivatives of natural bile acids, preferably 6-Ethyl-CDCA or taurine- or glycine-conjugated 6-Ethyl-CDCA, natural non-steroidal agonists, preferably Diterpenoids such as Cafestol and Kahweol, or synthetic non-steroidal FXR agonists.

In some embodiments, the FXR agonist is selected from the group consisting of obeticholic acid (Intercept Pharma), cholic acid (CT-RS); GS-9674 (Cilofexor) (Phenex Pharmaceuticals AG), Tropifexor (LJN452) (Novartis Pharmaceuticals), EYP001, EDP-305, a steroidal non-carboxylic acid FXR agonist (Enanta Pharmaceuticals), Turofexorate Isopropyl (Pfizer), INT-767 (Intercept Pharmaceuticals), LY-2562175 (Lilly), AGN-242266 (former AKN-083, Allergan), EP-024297 (Enanta Pharmaceuticals), M-480 (Metacrine), MET-409 (Metacrine), RDX-023 (Ardelyx), GW6046, Cafestol, Fexaramine and the compound PXL007 (also named EYP001 or EYP001a) identified by the CAS No. 1192171-69-9 (described in WO 2009127321). In a particular embodiment, the FXR agonist is selected from the group consisting of INT- 747, the compound identified by EDP-305 a steroidal non-carboxylic acid FXR agonist (Enanta Pharmaceuticals) and the compound identified by the CAS No. 1192171-69-9 (described in WO 2009127321).

In a particular aspect, the FXR agonist is selected from the group consisting of LJN452 (Tropifexor), GS-9674 (Cilofexor), LMB763 (Nidufexor), OCA (Ocaliva), EDP-305, TERN-001 and PXL007 (also named EYP001).

In a particular aspect, the FXR agonist is selected from the group consisting of the compound disclosed in Table 1.

**Table 1**

| | |
|---|---|
| UN452 (Tropifexor) | 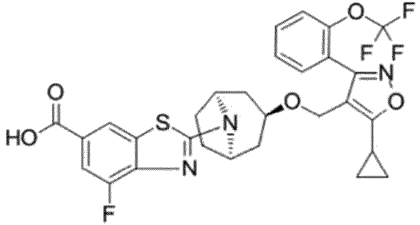 |
| Cas Number 1383816-29-2 | |
| 2-(3-((5-cyclopropyl-3-(2-(trifluoromethoxy)phenyl)isoxazol-4-yl)methoxy)-8-azabicyclo[3.2.1]octan-8-yl)-4-fluorobenzo[d]thiazole-6-carboxylic acid | |
| LMB763 (Nidufexor) | 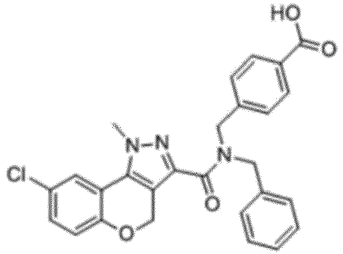 |
| Cas Number 1773489-72-7 | |
| 4-[(N-benzyl-8-chloro-1-methyl-1,4-dihydro[1]benzopyrano[4,3-c]pyrazole-3-carboxamido)methyl]benzoic acid | |
| GS-9674 (Cilofexor) | 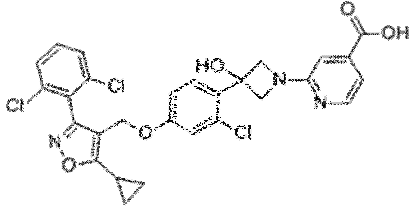 |
| Cas Number 1418274-28-8 | |
| 2-[3-[2-Chloro-4-[[5-cyclopropyl-3-(2,6-dichlorophenyl)-4-isoxazolyl]methoxy]phenyl]-3-hydroxy-1-azetidinyl]-4-pyridinecarboxylic acid | |
| PX-102 (PX-20606) | 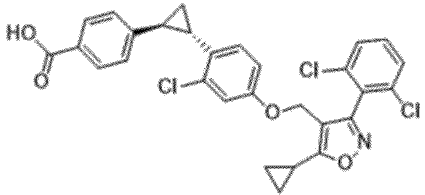 |
| Cas Number 1268244-85-4 | |
| 4-(2-(2-Chloro-4-((5-cyclopropyl-3-(2,6-dichlorophenyl)isoxazol-4-yl)methoxy)phenyl)cyclopropyl)benzoic acid | |
| PX-104 or Phenex 104 | enantiomer of PX-102 |
| OCA (Ocaliva or INT-747) | 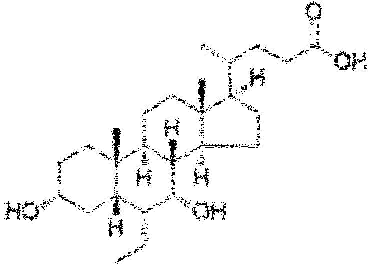 |
| Cas Number 459789-99-2 | |
| Cholan-24-oic acid, 6-ethyl-3,7-dihydroxy-, (3α,5β,6α,7α)- | |
| EDP-305 | 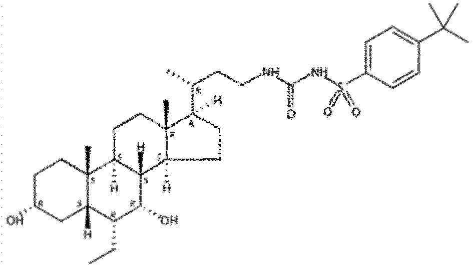 |
| Cas Number 1933507-63-1 | |
| Benzenesulfonamide, 4-(1,1-dimethylethyl)-N-[[[(3α,5β,6α,7α)-6-ethyl-3,7-dihydroxy-24-norcholan-23-yl]amino]carbonyl]- | |
| TERN-101(LY2562175) | 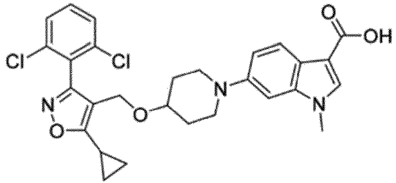 |
| Cas Number 1103500-20-4 | |
| 6-(4-{[5-Cyclopropyl-3-(2,6-dichlorophenyl)isoxazol-4-yl]methoxy}piperidin-1-yl)-1-methyl-1H-indole-3-carboxylic acid | |
| MET409 | Developed by Metacrine |
| | Disclosed in WO2017049173 |
| | 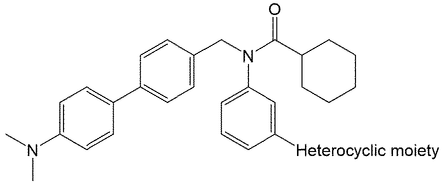 |
| GW4064 | 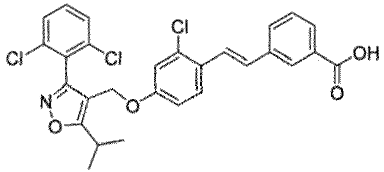 |
| Cas Number 278779-30-9 | |
| 3-[2-[2-Chloro-4-[[3-(2,6-dichlorophenyl)-5-(1-methylethyl)-4-isoxazolyl]methoxy]phenyl]ethenyl]benzoic acid | |
| WAY362450 (Turofexorate isopropyl or XL335 or FXR450) | 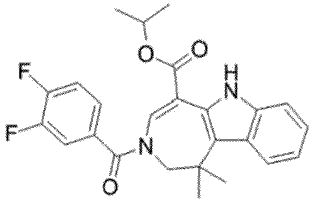 |
| Cas Number 629664-81-9 | |
| 3-(3,4-Difluoro-benzoyl)-1,1-dimethylene-1,2,3,6-tetrahydro-azepino [4,5-b]indole-5-carboxylic acid isopropyl ester, 3-(3,4-Difluorobenzoyl)-1,2,3,6-tetrahydro-1,1-dimethyl-azepino[4,5-b]indole-5-carboxylic acid 1-methylethyl ester, | |
| Fexaramine | 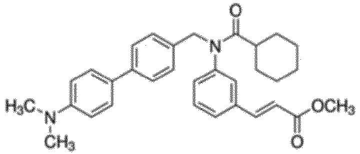 |
| Cas Number 574013-66-4 | |
| 3-[3-[(Cyclohexylcarbonyl)[[4'-(dimethylamino)[1,1'-biphenyl]-4-yl]methyl]amino]phenyl]-2-propenoic acid methyl ester | |
| AGN242266 (AKN-083) | 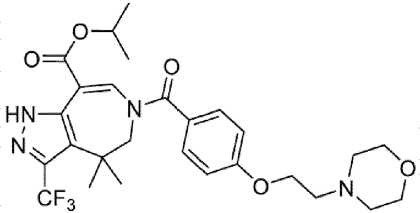 |
| BAR502 | 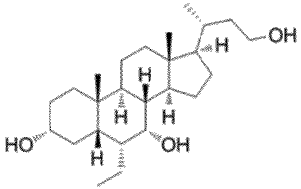 |
| Cas Number 1612191-86-2 | |
| 6α-ethyl-3α, 7α-dihydroxy-24-nor-5β-cholan-23-ol | |
| EYP001 | 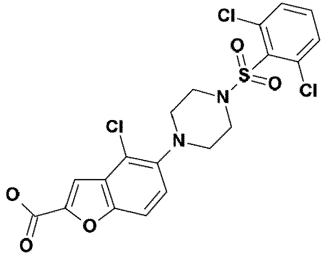 |
| Cas Number 1192171-69-9 | |

and any pharmaceutically acceptable salt thereof.

In a preferred aspect of the invention, the FXR agonist is EYP001.

Additional FXR agonists useful in the present inventions can be identified routinely by those of skill in the art based upon assays such as described in WO 2000/37077.

Typically, FXR agonists are identified using a nuclear receptor-peptide assay. This assay utilizes fluorescence resonance energy transfer (FRET) and can be used to test whether putative ligands bind to FXR. The FRET assay is based upon the principle that ligands induce conformational changes in nuclear receptors that facilitate interactions with coactivator proteins required for transcriptional activation. In FRET, a fluorescent donor molecule transfers energy via a non-radioactive dipole-dipole interaction to an acceptor molecule (which is usually a fluorescent molecule.

Typically, the FXR agonist of the invention is administered to the subject with a therapeutically effective amount. By a "therapeutically effective amount" of the FXR agonist as above described is meant a sufficient amount of the FXR agonist to treat a hepatitis D virus infection at a reasonable benefit/risk ratio applicable to any medical treatment. It will be understood, however, that the total daily usage of the compounds and compositions of the present invention will be decided by the attending physician within the scope of sound medical judgment. The specific therapeutically effective dose level for any particular patient will depend upon a variety of factors including the disorder being treated and the severity of the disorder; activity of the specific compound employed; the specific composition employed, the age, body weight, general health, sex and diet of the patient; the time of administration, route of administration, and rate of excretion of the specific compound employed; the duration of the treatment; drugs used in combination with the specific agonist employed; and like factors well known in the medical arts. For example, it is well known within the skill of the art to start doses of the compound at levels lower than those required to achieve the desired therapeutic effect and to gradually increase the dosage until the desired effect is achieved. However, the daily dosage of the products may be varied over a wide range from 0.01 to 1,000 mg per adult per day. Preferably, the compositions contain 0.01, 0.05, 0.1, 0.5, 1.0, 2.5, 5.0, 10.0, 15.0, 25.0, 50.0, 100, 250 and 500 mg of the active ingredient for the symptomatic adjustment of the dosage to the patient to be treated. A medicament typically contains from about 0.01 mg to about 500 mg of the active ingredient, preferably from 1 mg to about 100 mg of the active ingredient. An effective amount of the drug is ordinarily supplied at a dosage level from 0.0002 mg/kg to about 20 mg/kg of body weight per day, especially from about 0.001 mg/kg to 7 mg/kg of body weight per day.

### Combination therapy

According to an aspect of the invention, the FXR agonist according to the invention may be administered to the subject in combination with at least one other therapeutic agent, preferably in combination with at least one other antiviral agent, more preferably in combination with at least one other antiviral agent selected from the group consisting of immune system modulators, anti-HDV agents, anti-HBV agents, anti-HDV/HBV agents and any combination thereof. These agents are more particularly defined hereafter.

Accordingly, the present invention relates to
- a FXR agonist for the treatment of HDV infection, in particular a chronic HDV infection, in combination with at least one other therapeutic agent; or
- a pharmaceutical composition comprising a FXR agonist for the treatment of HDV infection, in particular a chronic HDV infection, in combination with at least one other therapeutic agent; or
- a pharmaceutical composition comprising a FXR agonist and at least one other therapeutic agent for the treatment of HDV infection, in particular a chronic HDV infection; or
- a kit comprising a FXR agonist and at least one other therapeutic agent as a combined preparation for simultaneous, separate or sequential use for the treatment of HDV infection, in particular a chronic HDV infection; or
- a method of treatment of a HDV infection in a subject, in particular a chronic HDV infection, comprising administering a therapeutically effective amount of a FXR agonist and a therapeutically effective amount of at least one other therapeutic agent; or
- a method of treatment of a HDV infection in a subject, in particular a chronic HDV infection, comprising administering a pharmaceutical composition comprising a therapeutically effective amount of a FXR agonist and a therapeutically effective amount of at least one other therapeutic agent.

The term "Immune system modulators" refers to signalling proteins of the interferon type (IFN), preferably to interferon alpha (IFN-α) or interferon lambda (IFN-λ), more preferably to pegylated interferon alpha (PEG-IFN-α) or pegylated interferon lambda (PEG-IFN-λ), and even more preferably to PEG-IFN-α2a, PEG-IFN-α2b or PEG-IFN-λ1a.

In one aspect, IFN is selected from the group consisting of consensus IFN-α (e.g., INFERGEN^{®}, Locteron^{®}), IFN-α1b (e.g., HAPGEN^{®}), IFN-α2a (Roferon-A^{®}, MOR-22, Inter 2A, Inmutag, Inferon), a pegylated IFN-α2a (e.g., PEGASYS^{®}, YPEG-IFNα-2a, PEG-INTRON^{®}, Pegaferon), IFN-α2b (e.g., INTRON A^{®}, Alfarona, Bioferon, Inter 2B, citpheron, Zavinex, Ganapar, etc...), a pegylated IFN-α2b (e.g., Pegintron ^{®}, Albuferon, AOP2014/P1101, Algeron, Pai Ge Bin), IFN-α2c (e.g. Berofor Alpha), and IFN-like protein (e.g., Novaferon, HSA-IFN-α2a fusion protein, HSA-IFN-α2b fusion protein).

IFN can be administered daily, weekly or 2, 3, 4, 5, or 6 times weekly. The treatment period is generally long, for instance from 2 weeks to several months. For instance, the period is from 3-4 months up to 24 months. The dosage can vary from 1 million units to 20 million units, for instance 2, 3, 4, 5, 6, 7, 8, 9, 10, 11, 12, 13, 14, 15, 16, 17, 18 or 19 million units. IFN can be administered by subcutaneous, intramuscular, intravenous, transdermal, or intratumoral administration, preferably for subcutaneous or intramuscular administration.

In a particular aspect, the IFN is used in combination with the FXR agonist at the beginning of the treatment. Optionally, the treatment with the IFN is stopped whereas the treatment with the FXR agonist is maintained. For instance, the first treatment period with the FXR agonist and the IFN may last several days or weeks (e.g., 1, 2, 3, 4, 5, 6, 7, 8 or 9 days or 1, 2, 3, 4, 5, 6, 7, 8 or 9 weeks) and is followed by a period of treatment with the FXR agonist in absence of IFN. This second step may last several days, weeks or months.

In a particular aspect, the IFN is IFNα2a, IFNα2b or a pegylated form thereof and is administered subcutaneously once a week, for instance at a dosage varying from 1 µg to 500 µg, preferably from 10 µg to 500 µg, more preferably from 100 µg to 250 µg, such as 100, 110, 120, 130, 140, 150, 160, 170, 180, 190 or 200 µg, and during from 2-4 months up to 24 months. In a very specific aspect, the treatment lasts from 12 to 52 weeks, preferably from 45 to 52 weeks, for instance 48 weeks. In a more specific aspect, the IFN is IFNα2a or a pegylated form thereof.

The term "anti-HDV agent" refers to any compound that treats HDV infection thereby inhibiting HDV replication, HDV virion assembly, or inhibiting HDV virion entry into infectable cells. Some anti-HDV agents are known by the person skilled in the art (see Deterding et al. 2019, AIDS Rev., 21, 126-134; Gilman et al. 2019), World J Gastroenterol., 25, 4580-4597). Preferably, the anti-HDV agent is selected from the group consisting of ribavirin, ritonavir, lonafarnib and EBP 921.

In particular, the inhibition of HDV replication, corresponds to a reduction of about 10, 20, 30, 40, 50, 60, 70, 80, 90% or 100 % of the number of HDV RNA copies of replicated in infected cells. Techniques for measuring the number of copies, particularly those based on Polymerase Chain Reaction (PCR), are well known to the person skilled in the art. Preferably, the anti-HDV agent that inhibits HDV replication is a nucleoside analog.

In particular, the inhibition of HDV virion assembly corresponds to a reduction of about 10, 20, 30, 40, 50, 60, 70, 80, 90 or 100 % of the amount of HDV virion assembled in infected cells. Techniques for quantifying the amount of virion, particularly those based on Enzyme-Linked Immunosorbent Assay (ELISA), are well known to the person skilled in the art. Preferably, the anti-HDV agent that inhibits HDV virion assembly is a farnesyl transferase inhibitor.

In particular, the inhibition of HDV virion entry into infectable cells correspond to a reduction of about 10, 20, 30, 40, 50, 60, 70, 80, 90 or 100 % of the amount of HDV virion entry in infectable cells.

The term "anti-HBV agent" refers to compounds that treat HBV infection thereby inhibiting HBV replication, inhibiting HBV virion assembly, or inhibiting HBV virion entry into infectable cells. Anti-HBV agent are known by the person skilled in the art, for example such as conventional interferon, pegylated interferon, nucleoside and nucleotide analogues (see Terrault *et* al. 2018). Preferably, the anti-HBV agent that inhibits HBV replication is a nucleoside analog, more preferably a nucleoside analog reverse-transcriptase inhibitor, and even more preferably, the anti-HBV agent is selected from the group consisting of lamivudine, adefovir, telbivudine, entecavir, tenofovir and emtricitabine.

In particular, the inhibition of HBV replication corresponds to a reduction of about 10, 20, 30, 40, 50, 60, 70, 80, 90% or 100 % of the amount of HBV DNA replicated in infected cells.

A lower level of replication of HBV DNA helps to reduce the level of HBV virion assembly which in turns induces a lower level of infection of other target cells. Therefore, it occurs a lower probability of providing HBV antigens for the assembly of HDV visions.

In particular, the inhibition of HBV virion assembly corresponds to a reduction of about 10, 20, 30, 40, 50, 60, 70, 80, 90 or 100 % of the amount of HBV virion assembled in infected cells.

A lower level of HBV virions assembly of HBV virions induces a lower level of infection of other target cells and therefore a lower probability of providing HBV antigens for the assembly of HDV visions.

In particular, the inhibition of HBV virion entry into infectable cells correspond to a reduction of about 10, 20, 30, 40, 50, 60, 70, 80, 90 or 100 % of the amount of HBV virion entry in infectable cells.

The terms "Infectable cells" refer to cells accessible to virions that express the NTCP receptor required for the HDV virion or HBV virion entry into the cell. A cell, particularly a host cell, is considered accessible when there is no biological or physical barrier to prevent its contact with the circulating virion.

Since HBV and HDV virions share the same entry receptor, i. e. NTCP, the docking-mediated blockage of NTCPs by anti-HBV agents effectively inhibiting cell entry of HBV virions, also inhibits cell entry of HDV virions.

The term "anti-HBV/HDV agent" refers to compounds that treat HDV and/or HBV infection thereby inhibiting HBV and HDV virion assembly, or inhibiting HBV and HDV virion entry into infectable cell. Preferably, the anti-HBV agent is selected from the group consisting of ezetimibe, myrcludex B, nucleic acid polymer REP 2139 and nucleic acid polymer REP 2165 or any combination thereof.

In particular, the inhibition of HBV and HDV virion assembly respectively correspond to a reduction of about 10, 20, 30, 40, 50, 60, 70, 80, 90 or 100 % of the amount of HBV virion or HDV virion assembled in infected cells. Preferably, the anti-HBV/HDV agent that inhibits HBV or HDV virion assembly is a nucleic acid polymer, more preferably a nucleic acid polymer that blocks the HBAgs secretion.

In particular, the inhibition of HBV virion and HDV virion entry into infectable cells correspond to a reduction of about 10, 20, 30, 40, 50, 60, 70, 80, 90 or 100 % of the amount of HBV virion or HDV virion entry in infectable cells. Preferably, the anti-HBV/HDV agent that inhibits HBV or HDV entry in infectable cells is a NTCP inhibitor, more preferably an NTCP-docking inhibitor.

According to a preferred aspect of the invention, the FXR agonist according to the invention may be administered to the subject in combination with at least one other antiviral agent selected from the group consisting of immune system modulators, anti-HDV agents, anti-HBV agents, anti-HDV/HBV agents as defined above and any combination thereof. Preferably said other antiviral agent is selected from the group consisting of immune system modulators of the interferon type, nucleoside analogues, nucleotide analogues, nucleic acid polymers, farnesyl transferase inhibitors, protease inhibitors, NTCP inhibitor and any combination thereof.

More preferably, the FXR agonist according to the invention may be administered to the subject in combination with PEG-IFN-α2a, PEG-IFN-α2b or PEG-IFN-λ1a, ribavirin, ritonavir, lonafarnib and EBP 921, lamivudine, adefovir, telbivudine, entecavir, tenofovir and emtricitabine, ezetimibe, myrcludex B, nucleic acid polymer REP 2139 and nucleic acid polymer REP 2165 and any combination thereof.

According to a specific aspect, the FXR agonist according to the invention may be administered to the subject in combination with PEG-IFN-α2a.

Alternatively, the FXR agonist according to the invention may be administered to the subject in combination with myrcludex B.

Alternatively, the FXR agonist according to the invention may be administered to the subject in combination with ritonavir.

Alternatively, the FXR agonist according to the invention may be administered to the subject in combination with lonafarnib.

Alternatively, the FXR agonist according to the invention may be administered to the subject in combination with adefovir.

Alternatively, the FXR agonist according to the invention may be administered to the subject in combination with PEG-IFN-α2a and another agent, preferably with PEG-IFN-α2a and myrcludex B. Alternatively, the FXR agonist according to the invention may be administered to the subject in combination with PEG-IFN-α2a and ritonavir.

Alternatively, the FXR agonist according to the invention may be administered to the subject in combination with PEG-IFN-α2a and lonafarnib.

Alternatively, the FXR agonist according to the invention may be administered to the subject in combination with PEG-IFN-α2a and adefovir.

In one aspect of the invention, the administration of the combination therapy is simultaneous, so that the FXR agonist and at least one other agent are simultaneously administered to the subject.

In another aspect of the invention, the administration of the combination therapy is sequential, so that the FXR agonist and at least one other agent are sequentially administered to the subject with a determined time delay, preferably about 1 to 10 days, more preferably about 1 to 24 hours, even more preferably of about 1 to 12 hours.

In a specific aspect, the FXR agonist is not used in combination with an interferon.

The invention will be further illustrated by the following figures and examples.

### FIGURES:

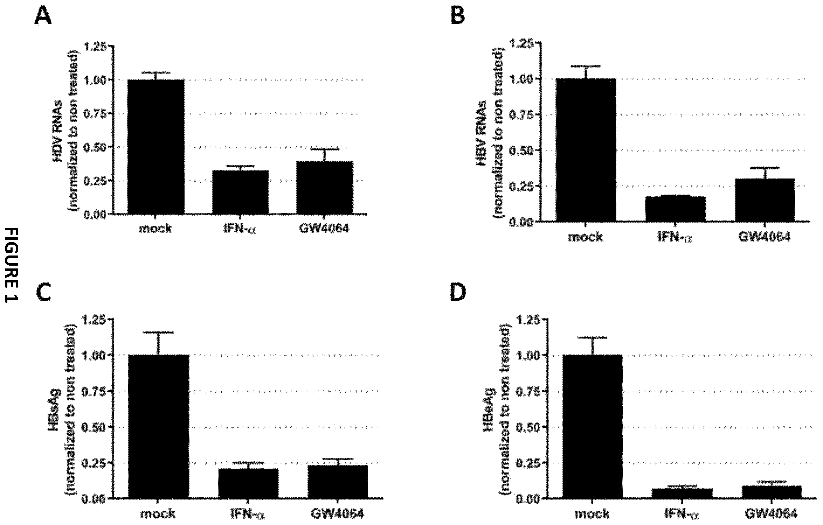

**Figure 1****.** *FXRα agonist inhibits HDV replication in HBV-HDV coinfected dHepaRG cells.* Differentiated HepaRG cells were infected with HBV at a MOI of 100 GE/cell and with HDV at a MOI of 10 GE/cell. From day 3 to 13 post-infection, cells were treated with 10 µM of GW4064, interferon α-2a (1000 IU/mL) or vehicle. Cells and supernatants were harvested at day 13 for intracellular HBV and HDV RNA and secreted antigens quantification. Results are the mean +/- SD of two experiments performed with three biological replicates.

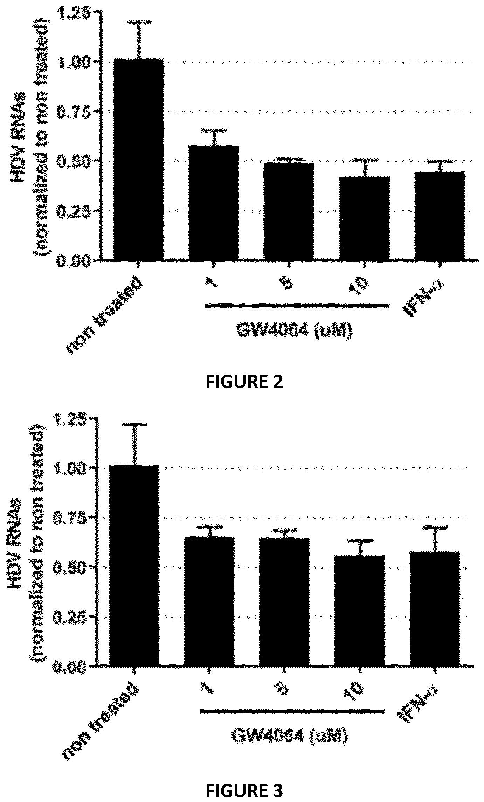

**Figure 2****.** *FXRα agonist inhibits HDV replication in HBV-infected dHepaRG super-infected with HDV.* Differentiated HepaRG cells were infected with HBV at a MOI of 100 GE/cell and 7 days later with HDV at a MOI of 10 GE/cell. From day 10 to day 17 post-HBV infection, cells were treated with 1, 5 or 10 µM of GW4064, interferon α-2a (1000 IU/mL) or vehicle. Cells and supernatants were harvested at day 17 for intracellular HDV RNA quantification. Results are the mean +/- SD of three experiments (in dHepaRG) performed with three biological replicates.
**Figure 3****.** *FXRα agonist inhibits HDV replication in HBV-infected PHH super-infected with HDV.* PHH were infected with HBV at a MOI of 100 GE/cell and 4 days later with HDV at a MOI of 10 GE/cell. From day 7 to day 14 post-HBV infection, cells were treated with 1, 5 or 10 µM of GW4064, interferon α-2a (1000 IU/mL) or vehicle. Cells and supernatants were harvested at day 14 post HBV infection for intracellular HDV RNA quantification. Results are the mean +/- SD of one experiment performed with three biological replicates.

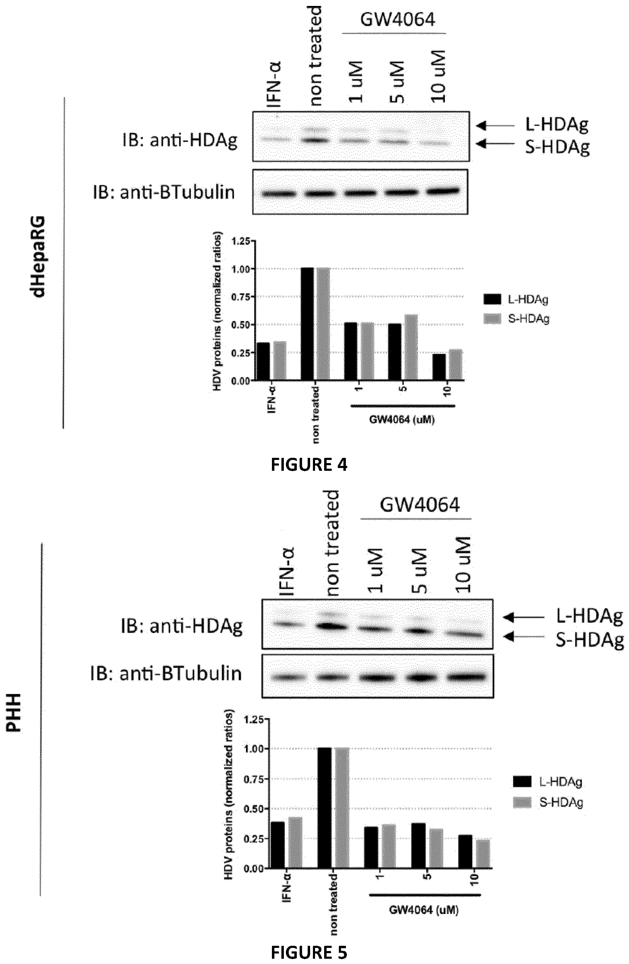

**Figure 4****.** *FXR α agonist inhibits the production of HDV proteins in HBV-infected dHepaRG superinfected with HDV.* Differentiated HepaRG cells were infected with HBV at a MOI of 100 GE/cell and 7 days later with HDV at a MOI of 10 GE/cell. From day 10 to day 17 post-HBV infection, cells were treated with 1, 5 or 10 µM of GW4064, interferon α-2a (1000 IU/mL) or vehicle. Cells were harvested at day 17 post HBV infection and lysed for protein extraction and WB analyses. Graphs represent the densitometry analyses of the respective blots and results are presented as ratios of HDAgs normalized to the levels of B-tubulin.
**Figure 5****.** *FXRα agonist inhibits the production of HDV proteins in HBV-infected PHH superinfected with HDV.* PHH were infected with HBV at a MOI of 100 GE/cell and 4 days later with HDV at a MOI of 10 GE/cell. From day 7 to day 14 post-HBV infection, cells were treated with 1, 5 or 10 µM of GW4064, interferon α-2a (1000 IU/mL) or vehicle. Cells and supernatants were harvested at day 14 post HBV infection for intracellular HDV RNA quantification and lysed for protein extraction and WB analyses. Graphs represent the densitometry analyses of the respective blots and results are presented as ratios of HDAgs normalized to the levels of B-tubulin.

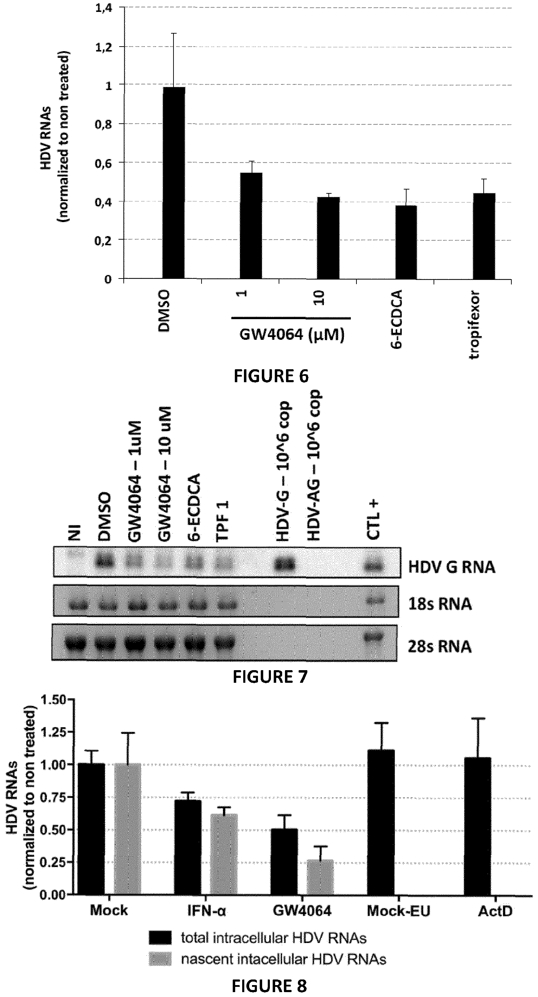

**Figure 6****.** *FXRα agonists inhibit HDV replication in monoinfected dHepaRG cells.* Differentiated HepaRG cells were infected with HDV at a MOI of 25 GE/cell. From day 4 to day 11 post-infection, cells were treated with 1 or 10 µM of GW4064, 10 µM of 6-ECDCA and 1 µM of tropifexor. At day 11 post HDV infection, cells were collected and total intracellular HDV RNAs were quantified by qPCR.
**Figure 7****.** *FXRα agonists decrease the amount of HDV genomic RNA in monoinfected dHepaRG cells.* Differentiated HepaRG cells were infected with HDV at a MOI of 25 GE/cell. From day 4 to day 11 post-infection, cells were treated with 1 or 10 µM of GW4064, 10 µM of 6-ECDCA and 1 µM of tropifexor. At day 11 post HDV infection, cells were collected and HDV genomic RNA was analyzed by Northern Blot.
**Figure 8****.** *Decrease of the levels of nascent HDV RNAs in HBV*/*HDV co-infected dHepaRG treated with FXRα agonists.* dHepaRG were co-infected with HBV (100 vge/cell) and with HDV (10 vge/cell). Six days later, cells were treated with GW4064 (10 µM) or with interferon α-2a (1000 U/mL) for 4 days. Cells were incubated with labelled uridin or not (mock-EU) for 2h, washed and harvested. Total intracellular HDV RNAs as well as EU-labelled HDV RNAs (nascent intracellular HDV RNAs) were isolated and quantified by RT-qPCR analyses. As a control, cells were treated with actinomycin D (10 µg/mL, ActD) 20 min before incubation with labelled uridin in order to block transcription of nascent RNAs. Results are the mean +/-SD one experiment performed with three biological replicates.

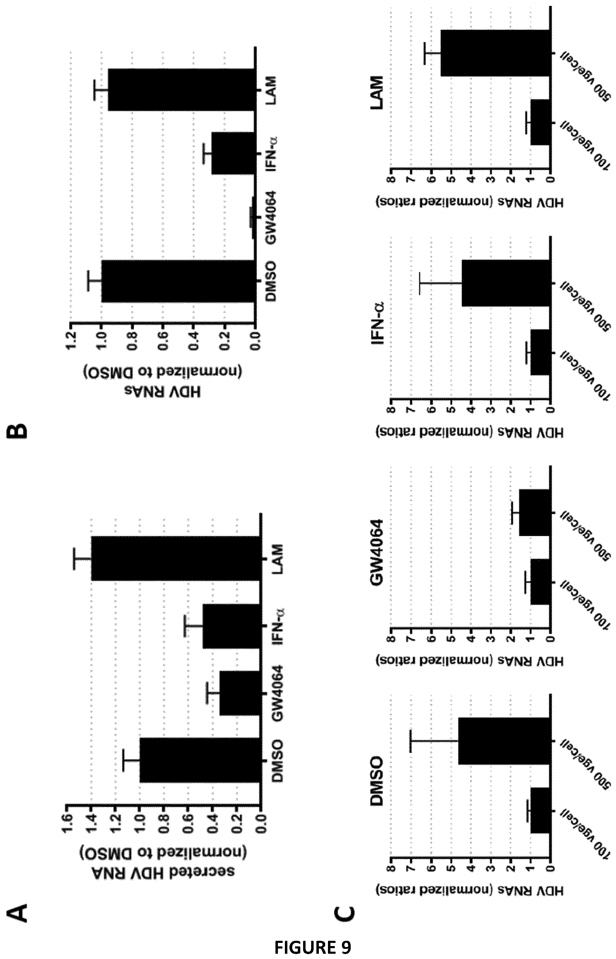

**Figure 9****. GW4064 reduces the infectivity of HDV particles.** dHepaRG cells were co-infected with HBV and HDV with 500 vge/cell for HBV and 50 vge/cell for HDV. Cells were treated or not 3 days later with GW4064 (10 µM), IFN-α (500 Ul/mL) or lamivudine (LAM, 10 µM) for 10 days. (A) Supernatants of infected dHepaRG cells were collected, concentrated by PEG precipitation and the levels of extracellular HDV RNAs were assessed by qRT-PCR analyses. (B-C) Naïve HuH7.5-^{NTCP} cells were infected with the different concentrated supernatants with (B) 500 vge/cell or (C) as indicated. Six days later, levels of intracellular HDV RNAs were assessed by RT-qPCR analyses. Results of RT-qPCR are the mean +/- SD of three independent experiments each performed with three biological replicates.

### EXAMPLES

### RESULTS

To determine the impact of FXR agonists on HDV infection, *in vitro* infections were performed in differentiated HepaRG cells (dHepaRG) and primary human hepatocytes (PHH).

After differentiation, HepaRG cells are susceptible to infection with HDV virions produced *in vitro,* either in monoinfection, or coinfection and superinfection with HBV. In the case of HBV/HDV coinfected or superinfected cells, this model allows the study of all steps of the HDV replication cycle, including penetration into the cell, translocation of the viral genome into the nucleus, replication of the viral genome and synthesis of viral mRNAs, as well as later stages of the viral cycle with assembly and secretion of infectious virions bearing HBV HBs envelope proteins. In HDV monoinfected cells, all steps of the viral cycle can be explored except the assembly process as newly-synthesized HBV envelope proteins are lacking.

PHH are also susceptible to infection with HDV virions produced *in vitro,* either in monoinfection, or coinfection and superinfection with HBV.

### Treatment with FXR modulators inhibit HDV replication in HBV/HDV coinfected HepaRG cells.

The inventors first evaluated the impact of FXRα agonists on HDV replication in *in vitro* coinfected dHepaRG cells. Cells were simultaneously infected with HBV and HDV. Three days post-infection, cells were treated for 10 days with FXR agonist GW4064 at 10 µM or 1000 IU/mL of interferon α-2a. At day 13 post-infection, cells and supernatants were collected. The intracellular amounts of HDV and HBV RNAs were quantified as well as secreted HBe and HBs antigens.

The amount of total intracellular HDV RNA was decreased by GW4064 by 60% in HepaRG at 10 µM (**Fig. 1A**). This decrease of viral RNA was comparable to that observed with interferon α-2a. The anti-HBV activity of GW4064, which was previously described, was verified on the amount of intracellular HBV RNAs and secreted HBs and HBe antigens (**Fig. 1B, 1C and 1D**).

### Treatment with FXR modulators inhibit HDV replication in HBV and HDV superinfected cells.

The impact of FXRα agonists on HDV replication was also evaluated in *in vitro* models of HDV superinfection of HBV-infected hepatocytes (both dHepaRG cells and PHH). Cells were successively infected with HBV and 7 days later with HDV. Three days post HDV infection, cells were treated for 7 days with GW4064 at 1, 5 and 10 µM or 1000 Ul/mL interferon α-2a. The intracellular amount of total HDV RNA was quantified by RT-qPCR.

In both dHepaRG cells and PHH, treatment with FXR agonist GW4064 decreased the amount of total intracellular HDV RNA, up to 60% in HepaRG cells (**Fig. 2**) and 45% in PHH (**Fig. 3**) at 10 µM. The effect was already very pronounced at 1 µM. This decrease of viral RNA was comparable to that observed with 1000 IU/mL of interferon α-2a.

Importantly, treatment with GW4064 also decreased the amount of HDV antigens (HDAg) in both superinfected dHepaRG cells (**Fig. 4**) and PHH (**Fig. 5**), as detected by Western Blot analysis. Of note, FXRα agonist decreased both HDAg-L (large HDV antigen) and HDAg-S (small HDV antigen) in the same proportions, *i.e.* 75% reduction of their amount in both models. Inhibition of HDAgs was slightly higher following treatment with 10µM of GW4064 than that obtained with 1000 IU/mL of interferon α-2a. Collectively these results indicate that FXRα agonist GW4064 likely inhibits HDV at the mRNA level, leading to a very strong inhibition at protein levels.

### Treatment with FXR modulators inhibit HDV replication in HDV monoinfected cells.

To determine if FXRα-mediated inhibition of HDV was independent of HBV, the inventors analyzed the impact of FXRα agonists in HDV mono-infected dHepaRG cells. Four days post-infection with HDV alone, cells were treated for seven days with 3 different FXRα agonists: 6-ECDCA at 10 µM, GW4064 at 1 and 10 µM and tropifexor at 1 µM. The impact of treatment on the amount of total HDV RNAs was analysed by RT-qPCR. The specific impact of FXR agonists on the amount of genomic HDV RNA was evaluated by Northern Blot analysis.

GW4064 at 10 µM, 6-ECDCA and tropifexor all decreased the amount of total HDV RNA by around 60%, as measured by RT-qPCR (**Fig. 6**) and also of genomic RNA detected by Northern Blot (**Fig. 7**).

Altogether these results indicate that FXRα agonists inhibit HDV infection in a way that is independent of their inhibitory effect on HBV infection. Moreover 3 different FXRα agonists with distinct structures showed comparable efficiency in HDV inhibition.

### Treatment with FXR modulators inhibit synthesis of nascent HDV RNAs.

To get initial insight on the mode of action of FWR agonists on HDV, the inventors performed Run-ON assay in HBV/HDV coinfected dHepaRG cells to determine whether nascent HDV RNA could be also inhibited as total HDV RNA amount was shown to be. dHepaRG cells were simultaneously infected with HBV and HDV. Six days post-infection, cells were treated with 10µM of GW4064 for 4 days before Run-On experiment. Results showed that GW4064 at 10µM was indeed able to inhibit the synthesis of HDV RNA within 2 hours of staining with labeled uridin, thus suggesting that the initiation of HDV mRNA and or elongation could be impacted (**Fig. 8**).

### Treatment with FXR modulators inhibit specific infectivity of secreted HDV viral particles.

The impact of FXRα agonists on secretion and specific infectivity of HDV viral particles was evaluated in *in vitro* coinfected dHepaRG cells. Three days post-infection, cells were treated for 10 days with FXR agonist GW4064 at 10 µM or 1000 IU/mL of interferon α-2a or lamivudine at 10 µM. Supernatants were collected and concentrated using 8 % PEG 8000. First, the secreted amounts of HDV RNA were quantified by RT-qPCR. Results showed that secretion of HDV RNA was decreased by GW4064 by 65 % at 10 µM (**Fig. 9A**). This decrease of viral RNA was slightly more pronounced to that observed with interferon α-2a (50%). As expected, HBV polymerase inhibitor lamivudine, used as control, did not significantly modify HDV RNA secretion.

Then, to determine the specific infectivity of secreted HDV particles, concentrated HDV viral particles collected from dHepaRG supernatants were used to infect naïve Huh7.5-^{NTCP} cells using the same vge/cell for each condition. Six days post infection, total intracellular HDV RNA was quantified by RT-qPCR. Results showed that, following infection with 500 vge/cell, the amount of intracellular HDV RNA was decreased by more than 95 % in cells infected by supernatants obtained from dHepaRG treated with FXR agonist GW4064 (**Fig. 9B**) compared to a 70 % decrease in the interferon α-2a condition. The specific infectivity of HDV particles was not modified by treatment with lamivudine.

Finally, naïve Huh7.5-^{NTCP} cells were infected with the same concentrated supernatants but using two different HDV inoculum, 100 and 500 vge/cell, for each condition. Quantification of intracellular HDV RNAs 6 days post infection showed a dose dependent increase of HDV RNA levels in cells infected with supernatants collected from dHepaRG treated with either vehicle, interferon α-2a or lamivudine (**Fig. 9C**). However, this was not the case using supernatants collected from dHepaRG cells treated with FXR agonist GW4064, as no significant difference was observed when naïve Huh7.5-^{NTCP} cells were infected with either 100 or 500 vge/cell. Overall, these results indicate that FXR agonist GW4064 severely decrease the infectious properties of secreted HDV particles.

### Conclusions

The inventors found that FXR agonists are inhibitors of HDV replication in dHepaRG and PHH, the two most relevant models for *in vitro* studies of HDV infection. This antiviral effect was demonstrated with three different FXR agonists, *i.e.* one bile acid analog (6-ECDCA) and 2 synthetic agonists (GW4064 and tropifexor).

The present results from experiments performed in HDV monoinfected cells clearly demonstrate that the inhibitory effect of FXR agonists on HDV replication is independent of the impact of this class of molecules on HBV, which has been previously identified. Whereas HDV depends on HBV surface proteins for entry into hepatocytes, the replication step of the viral cycle occurs independently on HBV. As the inventors observed that the amount of the genomic form HDV RNA as well as nascent RNAs both decreased following treatment, FXR agonists may target the replication step of HDV life cycle.

Moreover, the inventors showed that FXR agonists are also inhibitors of HDV secretion and specific infectivity of secreted viral particles in dHepaRG cells. This antiviral effect was demonstrated with synthetic agonist GW4064.

In conclusion, the inventors have identified new molecules (i.e. FXR agonists) that specifically regulate (inhibit) HDV infection. This should allow the selection of candidates who could be tested in an animal model or directly in humans with FXR agonists already in clinical trials.

### MATERIAL & METHODS

### Cell lines

### HepaRG

The HepaRG cell line derived from a human cellular hepato carcinoma can differentiate and regain many phenotypic traits of hepatocytes after 4 weeks of culture under defined conditions¹. HepaRG cells were cultured, differentiated, and infected by HBV and HDV as previously described^{2,3}. Briefly, for differentiation, cells were maintained for 2 weeks in standard medium then for at least 2 weeks in standard medium supplemented with 1,8% DMSO The composition of standard medium was the following: William's E medium supplemented with 10% HyCLone FetalClone II serum (Thermo Fisher Scientific), penicillin/streptomycin, L-glutamine, Insulin-Transferrin-Selenium (Gibco) and 50 µM hydrocortisone hemisuccinate.

### Primary human hepatocytes

Primary human hepatocytes (PHH) were freshly prepared from human liver resection obtained from the Centre Léon Bérard (Lyon) with French ministerial authorizations (AC 2013-1871, DC 2013 - 1870, AFNOR NF 96 900 sept 2011) as previously described⁴.

### Huh7.5^{NTCP}

Huh7.5 cells were kindly provided by C.M. Rice (Rockefeller University, USA). Derived Huh7.5^{NTCP} cells were generated by lentiviral transduction as previously described (Ni et al., Gastroenterology, 2014; 146(4):1070-83. doi: 10.1053/j.gastro.2013.12.024. Epub 2013 Dec 19. PMID: 24361467).

### Viruses

HDV stocks (genotype 1, Genbank ID M21012) were prepared from supernatants from co-transfected Huh7 cells as previously described^{3,5}. Plasmids pSVLD3 and pT7HB2.7 used for the production of infectious HDV particles have been kindly provided by Camille Sureau (Laboratoire de virologie moléculaire, Inserm UMR S_1134, Institut National de Transfusion Sanguine, Paris, France).

HBV stocks (genotype D, Genbank ID U95551) were prepared using the HepAD38 cell line according to previously described protocols⁷.

Supernatants containing HBV or HDV particles were clarified (0.45 µm filter) and concentrated with 8% PEG 8000 (Sigma-Aldrich).

HDV RNA was quantified by RT-qPCR as previously described⁶ and HBV DNA was quantified using the AmpliPrep/COBAS^{®} TaqMan^{®} HBV Test (Roche).

### Chemicals

GW4064 [3-(2,6-dichlorophenyl)-4-(3-carboxy-2-chloro-stilben-4-yl)-oxymethyl-5-isopropyl isoxazole] is a FXR agonist (EC50 90 nM), active both in vivo and in vitro⁸. Although displaying a limited bioavailability, GW4064 has gained a widespread use as a powerful and selective FXR agonist and has reached the status of "reference compound" in this field.

6-ECDCA (6-ethyl-cheno-deoxycholic acide) is a bile salt derivative and strong FXR agonist (EC50 99 nM) and was obtained from Sigma-Aldrich⁹.

Tropifexor (2-[(1R,3r,55)-3-({5-cyclopropyl-3-[2-(trifluoromethoxy)phenyl]-1,2-oxazol-4-yl}methoxy)-8-azabicyclo[3.2.1]octan-8-yl]-4-fluoro-1,3-benzothiazole-6-carboxylic acid) is a synthetic FXR agonist, active in vitro and in vivo, and was obtained from Cayman¹⁰.

GW4064, 6-ECDCA and tropifexor were all dissolved in DMSO at 10 mM to prepare stock solutions. Interferon alpha-2 (ROFERON-A) was purchased from Roche.

Actinomycin D was purchased from Sigma-Aldrich.

Lamivudine (LAM) was purchased from Selleckchem.

### Western Blot

Cells were harvested in RIPA lysis buffer (NaCl 150 mM, Tris HCl pH = 8,0 50 mM, SDS 0,1%, NP40 1%, Na Deoxycholate 0,5%) containing protease inhibitors (Protein Cocktail Inhibitors from Sigma-Aldrich, NaF 10 mM, Na Orthovanadate 10 mM). Clarified lysates were subjected to 10% SDS-PAGE and Western Blot transfer onto PVDF or nitrocellulose membranes using the TransTurbo Blot apparatus according to the manufacturer (Biorad). Primary antibodies are the HDVAg antibody (kind gift of Dr Alain Kay) and the beta-tubulin antibody (Abcam). Secondary HRP antibodies were purchased from Sigma-Aldrich. HRP signal detection was determined electronically using Ozyme - Syngene PXi Image system and parameters set strictly below the saturation point.

### Northern Blot

Total RNA was extracted from infected cells using Tri Reagent^{®} (TR118, Molecular Research Center). For each sample, 2 µg of total RNA were subjected to electrophoresis in gels of 1.2% agarose. After electrotransfer to charged nylon membranes (Roche), genomic HDV RNA sequences were detected by using a strand-specific RNA probe synthesized using the Dig RNA Labeling kit (Sp6/T7) (Roche) and DIG luminescent detection kit (Roche) accordinng to the manufacturer's instruction. Quantification of signals were done with ImageLab.

As an internal control for the amount and quality of the extracted RNA, the membrane was stripped and rehybridized by using labeled oligonucleotides specific for human 18S rRNA and 28S rRNA.

### HBs and HBe quantification

HBs and HBe antigens secreted in cells supernatant were quantified, after required dilutions, on Mini Vidas apparatus with Vidas HBs and Vidas HBE/HBET kits (bioMérieux, France) or Autobio kits (AutoBio, China) according to manufacturer's protocol.

### Quantification of viral RNAs by qPCR

Total RNA was prepared using NucleoSpin RNA Plus (Macherey-Nagel). After DNA digestion with TURBO DNase (Ambion), maximum 1000 ng RNA were reverse-transcribed using High-Capacity RNA-to-cDNA kit (Thermo Fisher Scientific). Quantitative PCR was carried out with primers HDV-F (5'-GCCTCTCCTTGTCGGTGAAT -3', SEQ ID NO: 1) and HDV-R (5'-CCTGGCTGGGGAACATCAAA-3', SEQ ID NO: 2) for quantification of total HDV RNA and HBV-F (5'-AGCTACTGTGGAGTTACTCTCGT-3', SEQ ID NO: 3) and HBV-R (5'-CAAAGAATTGCTTGCCTGAGTG-3', SEQ ID NO: 4) for quantification of pregenomic/precore HBV RNA. cDNA was analysed by quantitative PCR (qPCR) using QuantiFast SYBR^{®} Green PCR kit (Qiagen) on LightCycler^{®} 480 instrument (Roche) using a 45 PCR cycles. All assays were performed in triplicate. Relative quantification was determined by normalizing the expression of each gene to S9 housekeeping gene using primers S9-F (5'-CCGCGTGAAGAGGAAGAATG-3', SEQ ID NO: 5) and S9-R (5'-TTGGCAGGAAAACGAGACAAT-3', SEQ ID NO: 6).

### Run-On assays

HDV-infected HepaRG cells were incubated with labelled uridin or not (mock-EU) for 2h, washed and harvested. Total intracellular HDV RNAs as well as EU-labelled HDV RNAs (nascent intracellular HDV RNAs) were isolated using the Click-iT^{™} Nascent RNA Capture Kit (Thermofisher Scientific) according to the manufacturer's instruction. As a control, cells were treated with 10 µg/mL of actinomycin D 20 min before incubation with labelled uridin in order to block transcription of nascent RNAs.

### Analysis of HDV secretion and specific infectivity

For analysis of HDV virion specific infectivity, supernatants from dHepaRG infected with both HBV and HDV were concentrated using 8% PEG 8000. HDV RNA was quantified by RT-qPCR in concentrates and Huh7.5^{NTCP} cells were infected using same viral genome equivalents (vge) of concentrated virus for each condition of treatment. Six days post infection, total cellular RNA was extracted and HDV RNA was quantified by RT-qPCR.

### BIBLIOGRAPHY

1. Hantz, O. et al. J. Gen. Virol. 90, 127-135 (2009).
2. Gripon, P. et al. Proc. Natl. Acad. Sci. U. S. A. 99, 15655-15660 (2002).
3. Alfaiate, D. et al. Antiviral Res. 136, 19-31 (2016).
4. Lecluyse, E. L. & Alexandre, E. Methods Mol. Biol. Clifton NJ 640, 57-82 (2010).
5. Sureau, C. The Use of Hepatocytes to Investigate HDV Infection: The HDV/HepaRG Model. in Hepatocytes (ed. Maurel, P.) vol. 640 463-473 (Humana Press, 2010).
6. Scholtes, C. et al. J. Clin. Microbiol. 50, 2126-2128 (2012).
7. Ladner, S. K. et al. Antimicrob. Agents Chemother. 41, 1715-1720 (1997).
8. Maloney, P. R. et al. J. Med. Chem. 43, 2971-2974 (2000).
9. Pellicciari, R. et al. J. Med. Chem. 45, 3569-3572 (2002).
10. Tully, D. C. et al. J. Med. Chem. 60, 9960-9973 (2017).

## Claims

1. A farnesoid X receptor (FXR) agonist for use for the treatment of hepatitis D virus (HDV) infection in a subject in need thereof, wherein the subject suffers from a chronic HDV infection.

2. The FXR agonist for use of claim 1, wherein the FXR agonist is a selective FXR agonist.

3. The FXR agonist for use of any one of claims 1 to 2, wherein the FXR agonist is selected from the group consisting of UN452 (Tropifexor), LMB763 (Nidufexor), GS-9674 (Cilofexor), PX-102 (PX-20606), PX-104 (Phenex 104), OCA (Ocaliva), EDP-305, TERN-101 (LY2562175), MET-409, GW4064, WAY362450 (Turofexorate isopropyl), Fexaramine, AGN242266 (AKN-083), BAR502, and EYP001.

4. The FXR agonist for use of any one of claims 1 to 2, wherein the FXR agonist is EYP001.

5. The FXR agonist for use of any one of claims 1 to 4, for use in combination with an interferon alpha (IFN-α), an interferon lambda or a pegylated form thereof, preferably selected from the group consisting of IFN-α1a, IFN-α1b, IFN-α2a, IFN-α2b, and IFN-λ1a or a pegylated form thereof, more preferably PEG-IFN-α2a (e.g., Pegasys), PEG-IFN-α2b (e.g., ViraferonPeg or Introna) or PEG-IFN-λ1a.

6. The FXR agonist for use of any one of claims 1 to 5, for use in combination with an anti-HDV agent selected from the group consisting of ribavirin, ritonavir, lonafarnib and EBP 921.

7. The FXR agonist for use of any one of claims 1 to 6, for use in combination with an anti-HBV agent selected from the group consisting of lamivudine, adefovir, telbivudine, entecavir, tenofovir and emtricitabine.

8. The FXR agonist for use of any of claims 1 to 6, for use in combination with an anti-HBV/HDV agent selected from the group consisting of ezetimibe, myrcludex B, nucleic acid polymer REP 2139 and nucleic acid polymer REP 2165.

9. The FXR agonist for use of any one of claims 1 to 7, wherein the subject has failed to respond to a previous treatment for HDV infection.

10. The FXR agonist for use of claim 9, wherein the previous treatment is a treatment with PEG-IFNα.

11. The FXR agonist for use of claim 9, wherein the previous treatment is a treatment with an anti-HDV agent selected from the group consisting of ribavirin, ritonavir, lonafarnib and EBP 921.

## Patentansprüche

1. Farnesoid-X-Rezeptor (FXR)-Agonist zur Verwendung zur Behandlung einer Hepatitis-D-Virus (HDV)-Infektion bei einem Subjekt, das dies benötigt, wobei das Subjekt an einer chronischen HDV-Infektion leidet.

2. Der FXR-Agonist zur Verwendung nach Anspruch 1, wobei der FXR-Agonist ein selektiver FXR-Agonist ist,

3. Der FXR-Agonist zur Verwendung nach einem der Ansprüche 1 bis 2, wobei der FXR-Agonist ausgewählt ist aus der Gruppe bestehend aus LIN452 (Tropifexor), LMB763 (Nidufexor), GS-9674 (Cilofexor), PX-102 (PX- 20606), PX-104 (Phenex 104), OCA (Ocaliva), EDP-305, TERN-101 (LY2562175), MET-409, GW4064, WAY362450 (Turofexorat-Isopropyl), Fexaramine, AGN242266 (AKN-083), BAR502, und EYP001.

4. Der FXR-Agonist zur Verwendung nach einem der Ansprüche 1 bis 2, wobei der FXR-Agonist EYP001 ist.

5. FXR-Agonist zur Verwendung nach einem der Ansprüche 1 bis 4 zur Verwendung in Kombination mit einem Interferon alpha (IFNα-α), einem Interferon lambda oder einer pegylierten Form davon, vorzugsweise ausgewählt aus der Gruppe bestehend aus IFN-α1a, IFN- α1b, IFN- α2a, IFN- α2b und IFN-λ1a oder einer pegylierten Form davon, bevorzugter PEG-IFN- α2a (z. B. Pegasys), PEG-IFN- α2b (z. B. ViraferonPeg oder Introna) oder PEG-IFN- λ1a.

6. FXR-Agonist zur Verwendung nach einem der Ansprüche 1 bis 5 zur Verwendung in Kombination mit einem Anti-HDV-Mittel, ausgewählt aus der Gruppe bestehend aus Ribavirin, Ritonavir, Lonafarnib und EBP 921.

7. FXR-Agonist zur Verwendung nach einem der Ansprüche 1 bis 6 zur Verwendung in Kombination mit einem Anti-HBV-Mittel, ausgewählt aus der Gruppe bestehend aus Lamivudin, Adefovir, Telbivudin, Entecavir, Tenofovir und Emtricitabin.

8. FXR-Agonist zur Verwendung nach einem der Ansprüche 1 bis 6 zur Verwendung in Kombination mit einem Anti-HBV/HDV-Mittel, ausgewählt aus der Gruppe bestehend aus Ezetimib, Myrcludex B, Nukleinsäurepolymer REP 2139 und Nukleinsäurepolymer REP 2165

9. FXR-Agonist zur Verwendung nach einem der Ansprüche 1 bis 7, wobei das Subjekt auf eine frühere Behandlung einer HDV-Infektion nicht angesprochen hat

10. FXR-Agonist zur Verwendung nach Anspruch 9, wobei die vorherige Behandlung eine Behandlung mit PEG-IFNα ist.

11. FXR-Agonist zur Verwendung nach Anspruch 9, wobei die vorherige Behandlung eine Behandlung mit einem Anti-HDV-Mittel ist, ausgewählt aus der Gruppe bestehend aus Ribavirin, Ritonavir, Lonafarnib und EBP 921.

## Revendications

1. Agoniste du récepteur farnésoïde X (FXR) pour une utilisation dans le traitement d'une infection par un virus de l'hépatite D (VHD) chez un sujet en ayant besoin, dans lequel le sujet souffre d'une infection par VHD chronique.

2. Agoniste de FXR pour une utilisation selon la revendication 1, lequel agoniste de FXR est un agoniste de FXR sélectif.

3. Agoniste de FXR pour une utilisation selon l'une quelconque des revendications 1 et 2, lequel agoniste de FXR est choisi dans le groupe constitué par LIN452 (Tropifexor), LMB763 (Nidufexor), GS9674 (Cilofexor), PX-102 (PX-20606), PX-104 (Phenex 104), OCA (Ocaliva), EDP-305, TERN-101 (LY2562175), MET-409, GW4064, WAY362450 (Turofexorate isopropyle), la Fexaramine, AGN242266 (AKN-083), BAR502, et EYP001.

4. Agoniste de FXR pour une utilisation selon l'une quelconque des revendications 1 et 2, lequel agoniste de FXR est EYP001.

5. Agoniste de FXR pour une utilisation selon l'une quelconque des revendications 1 à 4, destiné à être utilisé en combinaison avec un interféron alpha (IFN-α), un interféron lambda ou une forme PEG-ylée de ceux-ci, de préférence choisi dans le groupe constitué par IFN-α1a, IFN-α1b, IFN-α2a, IFN-α2b, et IFN-λ1a ou une forme PEG-ylée de ceux-ci, mieux encore PEG-IFN-α2a (par exemple Pegasys), PEG-IFN-α2b (par exemple ViraferonPeg ou Introna), ou PEG-IFN-λ1a.

6. Agoniste de FXR pour une utilisation selon l'une quelconque des revendications 1 à 5, destiné à être utilisé en combinaison avec un agent anti-VHD choisi dans le groupe constitué par la ribavirine, le ritonavir, le lonafarnib et EBP 921.

7. Agoniste de FXR pour une utilisation selon l'une quelconque des revendications 1 à 6, destiné à être utilisé en combinaison avec un agent anti-VHB choisi dans le groupe constitué par la lamivudine, l'adéfovir, la telbivudine, l'entécavir, le ténofovir et l'emtricitabine,

8. Agoniste de FXR pour une utilisation selon l'une quelconque des revendications 1 à 6, destiné à être utilisé en combinaison avec un agent anti-VHB/anti-VHD choisi dans le groupe constitué par l'ézétimibe, le myrcludex B, le polymère d'acide nucléique REP 2139 et le polymère d'acide nucléique REP 2165.

9. Agoniste de FXR pour une utilisation selon l'une quelconque des revendications 1 à 7, dans lequel le sujet a échoué à répondre à un traitement antérieur pour une infection par VHD.

10. Agoniste de FXR pour une utilisation selon la revendication 9, dans lequel le traitement antérieur est un traitement avec du PEG-IFNα.

11. Agoniste de FXR pour une utilisation selon la revendication 9, dans lequel le traitement antérieur est un traitement avec un agent anti-VHD choisi dans le groupe constitué par la ribavirine, le ritonavir, le lonafarnib et EBP 921.
